(19) Europäisches Patentamt
European Patent Office
Office européen des brevets

(11) Numéro de publication : **0 408 418 B1**

(12) # FASCICULE DE BREVET EUROPEEN

(45) Date de publication du fascicule du brevet : **25.08.93 Bulletin 93/34**

(51) Int. Cl.$^5$ : **C07C 37/60,** C07C 39/08, C07C 41/26, C07C 43/23, B01J 27/16

(21) Numéro de dépôt : **90401882.7**

(22) Date de dépôt : **29.06.90**

(54) **Procédé d'hydroxylation de phénols et d'éthers de phénols.**

(30) Priorité : **11.07.89 FR 8909672**

(43) Date de publication de la demande : **16.01.91 Bulletin 91/03**

(45) Mention de la délivrance du brevet : **25.08.93 Bulletin 93/34**

(84) Etats contractants désignés : **AT BE CH DE DK ES FR GB GR IT LI LU NL SE**

(56) Documents cités :
**DE-B- 2 514 742**
**DE-B- 2 658 545**
**FR-A- 2 182 668**
**HOUBEN-WEYL: "METHODEN DER ORGANISCHEN CHEMIE", 4ième édition, vol. VI/Ic, pages 36, 37, Georg Thieme Verlag, Stuttgart, DE**

(73) Titulaire : **RHONE-POULENC CHIMIE**
**25, quai Paul Doumer**
**F-92408 Courbevoie Cédex (FR)**

(72) Inventeur : **Costantini, Michel**
**10 rue du Docteur Bonhomme**
**F-69003 Lyon (FR)**
Inventeur : **Laucher, Dominique**
**205 avenue Jean-Jaurès**
**F-69007 Lyon (FR)**

(74) Mandataire : **Dutruc-Rosset, Marie-Claude et al**
**RHONE-POULENC CHIMIE Service Brevets Chimie 25, Quai Paul Doumer**
**F-92408 Courbevoie Cédex (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



Jouve, 18, rue Saint-Denis, 75001 PARIS

## Description

La présente invention concerne l'hydroxylation de phénols et d'éthers de phénols par le peroxyde d'hydrogène.

Le brevet français n° 69.45467 publié sous le numéro 2 071 464 décrit un procédé d'hydroxylation de phénols et d'éthers de phénols par l'eau oxygénée, catalysé par un acide fort. Parmi ces acides forts l'acide sulfurique, l'acide paratoluènesulfonique, l'acide perchlorique sont les plus utilisés.

Le procédé selon ce brevet est un procédé industriel très important. Cependant la littérature fait état depuis quelques années de la recherche d'une catalyse de cette réaction, qui serait moins corrosive pour l'appareillage.

Ainsi la demande de brevet français publiée sous le numéro FR 2 489 816 préconise l'utilisation de silicalites de différents métaux.

La demande de brevet européen EP-A-0 299 893 décrit l'utilisation d'argiles pontées.

Bien que ces procédés utilisant une catalyse hétérogène paraissent intéressants, il semble que, d'une part il se pose encore des problèmes industriels tels que le recyclage du catalyseur et que, d'autre part, l'efficacité ne soit pas tout-à-fait aussi bonne qu'avec la catalyse par acide fort.

La présente invention permet d'obtenir des rendements d'hydroxylation aussi élevés qu'avec les acides forts, tout en limitant les risques de corrosion de l'appareillage.

Plus précisément l'invention consiste en un procédé d'hydroxylation de phénols ou d'éthers de phénols de formule générale (I) :

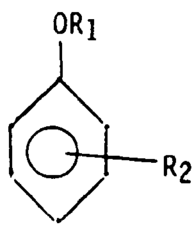

(I)

dans laquelle :

- $R_1$ représente un atome d'hydrogène, un groupement méthyle, un groupement éthyle ou un groupement phényle ;
- $R_2$ représente un atome d'hydrogène, un radical alkyle ayant de 1 à 4 atomes de carbone, un radical alcoxy ayant de 1 à 4 atomes de carbone, un radical phényle ou cyclohexyle,

par réaction avec le peroxyde d'hydrogène, caractérisé en ce que l'on opère en présence :

- d'une quantité efficace d'un sel de métal alcalin ou alcalino-terreux d'au moins un acide protonique ayant un pKa dans l'eau inférieur à - 0,1 ;
- et d'une quantité efficace d'au moins un oxacide du phosphore.

Les acides protoniques dont les sels servent de catalyseurs dans le présent procédé sont de préférence ceux dont le pKa dans l'eau est inférieur à -1.

Le pKa est défini comme la constante de dissociation ionique du couple acide/base, lorsque l'eau est utilisée comme solvant.

Parmi les sels des acides répondant à cette définition, il est préférable d'utiliser les sels de métaux alcalins ou alcalino-terreux des acides stables vis-à-vis de l'oxydation par le peroxyde d'hydrogène.

Ainsi, on peut citer plus particulièrement les sels de métaux alcalins ou alcalino-terreux de l'acide sulfurique, de l'acide pyrosulfurique, de l'acide perchlorique, de l'acide nitrique, des acides halogénosulfoniques tels que l'acide chlorosulfonique, l'acide fluorosulfonique, l'acide trifluorométhanesulfonique, l'acide méthanesulfonique, l'acide éthanesulfonique, l'acide éthanedisulfonique, l'acide benzènesulfonique, les acides benzènedisulfoniques, les acides toluènesulfoniques, les acides naphtalènesulfoniques et les acides naphtalènedisulfoniques.

Par sels de métaux alcalins, on entend dans le présent texte les sels neutres des acides définis précédemment de lithium, de sodium, de potassium, de rubidium et de césium.

On préfère le plus souvent utiliser les sels de sodium ou de potassium et encore plus préférentiellement, pour des raisons économiques, les sels de sodium.

Parmi ces différents sels, on peut citer parmi les préférés le sulfate disodique, le perchlorate de sodium, le trifluorométhanesulfonate de sodium, le paratoluènesulfonate de sodium, le chlorosulfonate de sodium, le fluorosulfonate de sodium, le méthanesulfonate de sodium.

Par sels de métaux alcalino-terreux, on entend dans le présent texte les sels neutres des acides définis précédemment de béryllium, de magnésium, de calcium, de strontium et de baryum.

On préfère le plus souvent utiliser les sels de magnésium, de calcium et de baryum.

Parmi ces différents sels de métaux alcalino-terreux, on mettra en oeuvre préférentiellement le sulfate de calcium, le sulfate de magnésium, le perchlorate de calcium, le perchlorate de magnésium, le trifluorométhanesulfonate de calcium, le trifluorométhanesulfonate de magnésium, le paratoluènesulfonate de calcium, le paratoluènesulfonate de magnésium, le chlorosulfonate de calcium, le chlorosulfonate de magnesium le fluorosulfonate de calcium, le fluorosulfonate de magnésium, le méthanesulfonate de calcium, le méthanesulfonate de magnésium.

On peut utiliser des mélanges de plusieurs sels de métaux alcalins ou alcalino-terreux.

On peut également préparer in-situ les sels de métaux alcalins ou alcalino-terreux par exemple en chargeant des quantités stoechiométriques d'acide et d'oxyde ou hydroxyde de ces métaux.

Les oxacides du phosphore sont plus particulièrement les composés à fonction acide du phosphore au degré d'oxydation 5.

On peut également utiliser des composés à fonction acide du phosphore au degré d'oxydation 3, qui seront oxydés dans le milieu par le peroxyde d'hydrogène en composés correspondants du phosphore V ; mais cela ne présente pas d'intérêt particulier, tout en ayant l'inconvénient de consommer une partie du peroxyde d'hydrogène.

Parmi ces oxacides du phosphore V, on peut citer par exemple l'acide orthophosphorique, l'acide métaphosphorique, l'acide pyrophosphorique, les acides polyphosphoriques, les acides phosphoniques tels que l'acide (hydroxy-1 éthylidène)diphosphonique, l'acide phosphonique, l'acide éthylphosphonique, l'acide phénylphosphonique.

Les plus couramment utilisés sont, pour des questions pratiques et économiques, l'acide orthophosphorique, l'acide pyrophosphorique et l'acide (hydroxy-1 éthylidène)diphosphonique.

La quantité de sel de métal alcalin ou alcalino-terreux utilisé dans le procédé de l'invention peut varier dans de larges limites.

Généralement cette quantité est exprimée en rapport molaire sel de métal alcalin ou alcalino-terreux/peroxyde d'hydrogène. Ce rapport est le plus souvent compris entre 0,1 % et 10 % en moles et de préférence entre 0,5 % et 5 % en moles.

La quantité d'oxacide du phosphore exprimée en rapport molaire oxacide du phosphore/peroxyde d'hydrogène est le plus souvent comprise entre 0,1 % et 20 % en moles et de préférence entre 0,5 % et 10 %.

Le peroxyde d'hydrogène peut être mis en oeuvre sous forme de solution aqueuse ou de solution organique.

Les solutions aqueuses étant commercialement plus facilement disponibles sont utilisées de préférence. Généralement elles contiennent plus de 20 % en poids de peroxyde d'hydrogène.

La quantité de peroxyde d'hydrogène peut aller jusqu'à 1 mole de $H_2O_2$ pour 1 mole de composé phénolique de formule (I).

Il est cependant préférable pour obtenir des rendements industriellement acceptables d'utiliser un rapport molaire composé phénolique de formule (I)/peroxyde d'hydrogène de 25/1 à 3/1 et de préférence de 20/1 à 4/1.

Afin d'avoir une vitesse de réaction suffisante, on limite la teneur initiale du milieu en eau à 20 % en poids et de préférence à 10 % en poids. Cette eau initiale correspond à l'eau introduite avec les réactifs et notamment avec le peroxyde d'hydrogène.

Parmi les composés phénoliques de formule (I) qui pourront être mis en oeuvre dans le procédé de l'invention, on peut citer à titre non limitatif le phénol, l'anisole, l'orthocrésol, le paracrésol, le métacrésol, le tertiobutyl-4 phénol, le méthoxy-2 phénol, le méthoxy-4 phénol.

Le présent procédé convient tout particulièrement bien à la préparation d'hydroquinone et de pyrocatéchol à partir du phénol.

La température à laquelle est conduite la réaction d'hydroxylation est généralement comprise entre 45°C et 160°C sous pression atmosphérique.

On peut également opérer à des températures plus élevées sous une pression supérieure à la pression atmosphérique.

Les réactifs et les conditions opératoires conviennent bien à une mise en oeuvre en continu du procédé de l'invention.

Les exemples qui suivent illustrent l'invention.

EXEMPLE 1

Dans un ballon en verre de 100 cm3, muni d'un réfrigérant ascendant, d'un thermomètre, d'une ampoule de coulée et d'une agitation centrale on charge :

- 47 g (0,5 mol) de phénol
- 0,137 g (0,615 mmol) de Mg $(ClO_4)_2$ : 1,23 % en mol par rapport au peroxyde d'hydrogène
- 0,053 g (0,3 mmol) d'acide pyrophosphorique : 0,6 % en mol par rapport au peroxyde d'hydrogène.

Après avoir porté ce mélange à 75°C sous agitation, on introduit 2,44 g d'une solution aqueuse de peroxyde d'hydrogène à 69,5 % en poids par poids (0,05 mol).

Après 3 heures de chauffage à 75°C, on dose par iodométrie le peroxyde d'hydrogène restant et par chromatographie liquide haute performance les diphénols formés.

On obtient les résultats suivants :

- taux de transformation (TT) de $H_2O_2$ 100 %
- rendement en hydroquinone par rapport à $H_2O_2$ transformé 28,5 %
- rendement en pyrocatéchol par rapport à $H_2O_2$ transformé 42,0 %
- rendement total en diphénols 70,5 %

EXEMPLE 2

Dans un ballon en verre de 100 cm3, muni d'un réfrigérant ascendant, d'un thermomètre, d'une ampoule de coulée et d'une agitation centrale on charge :

- 47 g (0,5 mol) de phénol
- 0,271 g (1,215 mmol) de Mg $(ClO_4)_2$ : 2,43 % en mol par rapport au peroxyde d'hydrogène
- 0,107 g (0,6 mmol) d'acide pyrophosphorique : 1,2 % en mol par rapport au peroxyde d'hydrogène.

Après avoir porté ce mélange à 75°C sous agitation, on introduit 2,44 g d'une solution aqueuse de peroxyde d'hydrogène à 69,5 % en poids par poids (0,05 mol).

Après 2 heures 20 de chauffage à 75°C, on dose par iodométrie le peroxyde d'hydrogène restant et par chromatographie liquide haute performance les diphénols formés.

On obtient les résultats suivants :

- taux de transformation (TT) de $H_2O_2$ 100 %
- rendement en hydroquinone par rapport à $H_2O_2$ transformé (RT) 29,0 %
- rendement en pyrocatéchol par rapport à $H_2O_2$ transformé (RT) 42,0 %
- rendement total en diphénols 71,0 %

EXEMPLE 3

On répète l'exemple 1 en remplaçant l'acide pyrophosphorique par 0,45 mmol d'acide orthophosphorique (0,9 % en moles par rapport au peroxyde d'hydrogène).

Après 5 heures 25 de réaction à 75°C on obtient les résultats suivants :

- TT de $H_2O_2$ 97,0 %
- RT en hydroquinone 23,0 %
- RT en pyrocatéchol 36,0 %
- RT total en diphénols 59,0 %

EXEMPLE 4

On répète l'exemple 1 en remplaçant l'acide pyrophosphorique par 0,3 mmol d'acide (hydroxy-1 éthylidène)diphosphonique (0,6 % en moles par rapport au peroxyde d'hydrogène).

Après 3 heures 25 de réaction à 75°C on obtient les résultats suivants :

- TT de $H_2O_2$ 100 %
- RT en hydroquinone 30,0 %
- RT en pyrocatéchol 41,5 %
- RT total en diphénols 71,5 %

ESSAI COMPARATIF A

On répète l'exemple 1, mais en omettant le sel de magnésium.

Après 5 heures 15 de réaction à 75°C on obtient les résultats suivants :

- TT de $H_2O_2$ 16,5 %
- RT en hydroquinone 16,5 %
- RT en pyrocatéchol 38,5 %
- RT total en diphénols 55,0 %

En l'absence de sel de métal alcalin ou alcalino-terreux, on observe un très faible taux de transformation de $H_2O_2$, malgré une durée de réaction plus longue que dans l'exemple 1.

ESSAI COMPARATIF B

On répète l'exemple 2, mais en omettant l'acide pyrophosphorique.

Après 4 heures 25 de réaction à 75°C on obtient les résultats suivants :

- TT de $H_2O_2$ 52,5 %
- RT en hydroquinone 13,5 %
- RT en pyrocatéchol 29,5 %
- RT total en diphénols 43,0 %

En l'absence d'oxacide du phosphore, on observe un taux de transformation de $H_2O_2$ plus faible, malgré une durée de réaction plus longue, ainsi qu'un rendement en diphénols plus faible, que dans l'exemple 2.

ESSAI COMPARATIF C

On répète l'exemple 2, mais en omettant le sel de magnésium.

La quantité de peroxyde d'hydrogène à 69,5 % en poids/poids chargée est de 1,31 g (26,8 mmol) : le rapport molaire phénol/$H_2O_2$ est donc de 18,6.

Après 2 heures de réaction à 150°C on obtient les résultats suivants :

- TT de $H_2O_2$ 99,5 %
- RT en hydroquinone 19,5 %
- RT en pyrocatéchol 35,0 %
- RT total en diphénols 54,5 %

En l'absence de sel de métal alcalin ou alcalino-terreux, on observe qu'il est nécessaire d'opérer à beaucoup plus haute température pour obtenir un taux de transformation de $H_2O_2$ équivalent à celui de l'exemple 2, (alors que le rapport initial $H_2O_2$/phénol était plus élevé) tout en ayant un rendement en diphénols plus faible.

ESSAI COMPARATIF D

Dans un ballon en verre de 100 cm3, muni d'un réfrigérant ascendant, d'un thermomètre, d'une ampoule de coulée et d'une agitation centrale on charge :

- 51,5 g (0,548 mol) de phénol
- 0,0981 g (0,68 mmol) de $HClO_4$ : 1,23 % en mol par rapport au peroxyde d'hydrogène.
- 0,058 g (0,33 mmol) d'acide pyrophosphorique : 0,6 % en mol par rapport au peroxyde d'hydrogène.

Après avoir porté ce mélange à 75°C sous agitation, on introduit 2,70 g d'une solution aqueuse de peroxyde d'hydrogène à 69,5 % en poids par poids (55,2 mmol).

Après 2 heures de chauffage à 75°C, on dose par iodométrie le peroxyde d'hydrogène restant et par chromatographie liquide haute performance les diphénols formés.

On obtient les résultats suivants :

- taux de transformation (TT) de $H_2O_2$ 99,5 %
- rendement en hydroquinone par rapport à $H_2O_2$ transformé 30,0 %
- rendement en pyrocatéchol par rapport à $H_2O_2$ transformé 42,0 %
- rendement total en diphénols 72,0 %

ESSAI COMPARATIF E

Dans un ballon en verre de 100 cm3, muni d'un réfrigérant ascendant, d'un thermomètre, d'une ampoule de coulée et d'une agitation centrale on charge :

- 58,1 g (0,618 mol) de phénol
- 0,222 g (1,58 mmol) d'acétate de Mg : 2,53 % en mol par rapport au peroxyde d'hydrogène
- 0,074 g (0,42 mmol) d'acide pyrophosphorique : 0,67 % en mol par rapport au peroxyde d'hydrogène.

Après avoir porté ce mélange à 75°C sous agitation, on introduit 3,05 g d'une solution aqueuse de peroxyde d'hydrogène à 69,5 % en poids par poids (62,4 mmol).

Après 2 heures 10 de chauffage à 75°C, on dose par iodométrie le peroxyde d'hydrogène restant et par chromatographie liquide haute performance les diphénols formés.

On obtient les résultats suivants :

- taux de transformation (TT) de $H_2O_2$ 7,5 %
- rendement en hydroquinine par rapport à $H_2O_2$ transformé 8,0 %
- rendement en pyrocatéchol par rapport à $H_2O_2$ transformé 15,0 %
- rendement total en diphénols 23,0 %

EXEMPLES 5 à 11

Dans l'appareil décrit à l'exemple 1, on charge :

- 47,0 g (0,5 mol) de phénol
- $NaClO_4,H_2O$ : % en moles par rapport au peroxyde d'hydrogène indiqué dans le tableau 1
- acide pyrophosphorique : % en moles par rapport au peroxyde d'hydrogène indiqué dans le tableau 1.

Après avoir porté ce mélange à 75°C ou 100°C sous agitation, on introduit des quantités variables d'une solution aqueuse de peroxyde d'hydrogène à 69,5 % en poids par poids (voir rapports molaires $H_2O_2$/phénol dans tableau 1).

Après des durées de chauffage variables à 75°C ou 100°C (voir tableau 1), on obtient les résultats indiqués dans le tableau 1 ci-après.

Les abréviations suivantes ont été utilisées :

- TT % = taux de transformation %
- RT HQ = rendement en hydroquinone par rapport à $H_2O_2$ transformé
- RT PC = rendement en pyrocatéchol par rapport à $H_2O_2$ transformé
- RT total = rendement total en diphénols.

TABLEAU 1

| Exemples | $H_2O_2$/Phénol (% en moles) | $NaClO_4/H_2O_2$ (% en moles) | $H_4P_2O_7/H_2O_2$ (% en moles) | T °C | Durée | TT % $H_2O_2$ | RT % HQ | RT % PC | RT % total |
|---|---|---|---|---|---|---|---|---|---|
| Ex. 5 | 5,7 | 2,34 | 1,1 | 100 | 4 h | 100 | 27,0 | 42,5 | 69,5 |
| Ex. 6 | 5,3 | 2,35 | 5,3 | 75 | 4h15 | 98,5 | 33,5 | 50,5 | 84,0 |
| Ex. 7 | 10,1 | 3,6 | 0,7 | 75 | 5h30 | 77,0 | 27,0 | 41,0 | 68,0 |
| Ex. 8 | 10,0 | 1,26 | 2,7 | 75 | 7 h | 92,0 | 28,0 | 43,5 | 71,5 |
| Ex. 9 | 10,4 | 1,75 | 5,1 | 75 | 6 h | 100 | 27,0 | 41,5 | 68,5 |
| Ex. 10 | 5,45 | 1,15 | 2,61 | 75 | 6 h | 97,0 | 34,0 | 47,0 | 81,0 |
| Ex. 11 | 5,13 | 1,27 | 2,66 | 100 | 4 h | 99,0 | 33,0 | 49,0 | 82,0 |

EXEMPLE 12 et ESSAI COMPARATIF F

Exemple 12

Dans l'appareil décrit à l'exemple 1, on charge :

- 42,9 g (0,456 mol) de phénol
- 0,285 g (1,16 mmol) de $MgSO_4,7H_20$ : 2,56 % en moles par rapport au peroxyde d'hydrogène
- 0,0746 g (0,42 mmol) d'acide pyrophosphorique : 0,93 % en moles par rapport au peroxyde d'hydrogène.

Après avoir porté ce mélange à 75°C sous agitation, on introduit 2,21 g d'une solution aqueuse de peroxyde d'hydrogène à 69,5 % en poids par poids (45,2 mmol).

Après 6 heures de réaction à 75°C on obtient les résultats suivants :

- TT de $H_2O_2$ 73,0 %
- RT en hydroquinone 23,5 %
- RT en pyrocatéchol 46,5 %
- RT total en diphénols 70,0 %

Essai comparatif F

On répète l'essai en l'absence d'acide pyrophosphorique.

On obtient les résultats suivants :

- TT de $H_2O_2$ 6,3 %
- RT en hydroquinone 15,9 %
- RT en pyrocatéchol 28,8 %
- RT total en diphénols 44,7 %

En l'absence d'oxacide du phosphore, on observe un taux de transformation de $H_2O_2$ très faible, ainsi qu'un rendement en diphénols plus faible, que dans l'exemple 12.

EXEMPLE 13

Dans l'appareil décrit à l'exemple 1, on charge :

- 44,5 g (0,473 mol) de phénol
- 0,153 g (1,12 mmol) de $CaSO_4$ : 2,28 % en moles par rapport au peroxyde d'hydrogène
- 0,0517 g (0,29 mmol) d'acide pyrophosphorique : 0,6 % en moles par rapport au peroxyde d'hydrogène.

Après avoir porté ce mélange à 75°C sous agitation, on introduit 2,40 g d'une solution aqueuse de peroxyde d'hydrogène à 69,5 % en poids par poids (49 mmol).

Après 4 heures 30 de réaction à 75°C on obtient les résultats suivants :

- TT de $H_2O_2$ 23,0 %
- RT en hydroquinone 21,0 %
- RT en pyrocatéchol 40,0 %
- RT total en diphénols 61,0 %

EXEMPLE 14

Dans l'appareil décrit à l'exemple 1, on charge :

- 39,4 g (0,418 mol) de phénol
- 0,086 g (0,55 mmol) de trifluorosulfonate de lithium : 1,23% en moles par rapport au peroxyde d'hydrogène
- 0,047 g (0,26 mmol) d'acide pyrophosphorique : 0,6 % en moles par rapport au peroxyde d'hydrogène.

Après avoir porté ce mélange à 75°C sous agitation, on introduit 2,07 g d'une solution aqueuse de peroxyde d'hydrogène à 69,5 % en poids par poids (42,3 mmol).

Après 6 heures de réaction à 75°C on obtient les résultats suivants :

- TT de $H_2O_2$ 81,5 %
- RT en hydroquinone 26,0 %
- RT en pyrocatéchol 41,5 %
- RT total en diphénols 67,5 %

EXEMPLE 15

Dans un réacteur de 30 cm3, muni d'un réfrigérant ascendant relié à un gazomètre, d'un thermomètre, d'un système de chauffage régulé, d'un système d'injection et d'une agitation centrale par barreaux aimantés, on charge :

- 9,4 g (0,1 mol) de phénol
- 0,137 g (0,615 mmol) de $Cs_2 SO_4$ : 2,5 % en mol par rapport au peroxyde d'hydrogène

- 0,011 g (0,06 mmol) d'acide pyrophosphorique : 0,6 % en mol par rapport au peroxyde d'hydrogène.

Après avoir porté ce mélange à 75°C sous agitation, on introduit 0,49 g d'une solution aqueuse de peroxyde d'hydrogène à 69,5 % en poids par poids (10 mmol).

Après 4 heures de réaction à 75°C on obtient les résultats suivants :

- rendement en hydroquinone par rapport à $H_2O_2$ chargé 16,5 %
- rendement en pyrocatéchol par rapport à $H_2O_2$ chargé 39,5 %
- rendement total en diphénols 56,0 %

## Revendications

1. Procédé d'hydroxylation de phénols ou d'éthers de phénols de formule générale (I) :

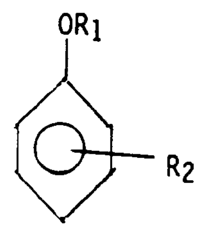

(I)

dans laquelle :
- $R_1$ représente un atome d'hydrogène, un groupement méthyle, un groupement éthyle ou un groupement phényle ;
- $R_2$ représente un atome d'hydrogène, un radical alkyle ayant de 1 à 4 atomes de carbone, un radical alcoxy ayant de 1 à 4 atomes de carbone, un radical phényle ou cyclohexyle,

par réaction avec le peroxyde d'hydrogène, caractérisé en ce que l'on opère en présence :
- d'une quantité efficace d'un sel de métal alcalin ou alcalino-terreux d'au moins un acide protonique ayant un pKa dans l'eau inférieur à - 0,1 ;
- et d'une quantité efficace d'au moins un oxacide du phosphore.

2. Procédé selon la revendication 1, caractérisé en ce que les acides protoniques dont les sels servent de catalyseurs sont ceux dont le pka dans l'eau est inférieur à -1.

3. Procédé selon l'une des revendications 1 ou 2, caractérisé en ce que l'on utilise les sels de métaux alcalins ou alcalino-terreux de l'acide sulfurique, de l'acide pyrosulfurique, de l'acide perchlorique, de l'acide nitrique, des acides halogénosulfoniques tels que l'acide chlorosulfonique, l'acide fluorosulfonique, l'acide trifluorométhanesulfonique, l'acide méthanesulfonique, l'acide éthanesulfonique, l'acide éthanedisulfonique, l'acide benzènesulfonique, les acides benzènedisulfoniques, les acides toluènesulfoniques, les acides naphtalènesulfoniques et les acides naphtalènedisulfoniques.

4. Procédé selon l'une des revendications 1 à 3, caractérisé en ce que l'on utilise comme sels de métaux alcalins les sels neutres des acides définis dans la revendication 3 de lithium, de sodium, de potassium, de rubidium et de césium et de préférence les sels de sodium ou de potassium.

5. Procédé selon l'une des revendications 1 à 4, caractérisé en ce que l'on utilise comme sels de métaux alcalins le sulfate disodique, le perchlorate de sodium, le trifluorométhanesulfonate de sodium, le paratoluènesulfonate de sodium, le chlorosulfonate de sodium, le fluorosulfonate de sodium, le méthanesulfonate de sodium.

6. Procédé selon l'une des revendications 1 à 3, caractérisé en ce que l'on utilise comme sels de métaux alcalino-terreux, les sels neutres des acides définis dans la revendication 3 de béryllium, de magnésium, de calcium, de strontium et de baryum et de préférence les sels de magnésium, de calcium et de baryum.

7. Procédé selon l'une des revendications 1 à 3 et 6, caractérisé en ce que l'on utilise comme sels de métaux alcalinoterreux, le sulfate de calcium, le sulfate de magnésium, le perchlorate de calcium, le perchlorate de magnésium, le trifluorométhanesulfonate de calcium, le trifluorométhanesulfonate de magnésium, le paratoluènesulfonate de calcium, le paratoluènesulfonate de magnésium, le chlorosulfonate de calcium,

le chlorosulfonate de magnésium, le fluorosulfonate de calcium, le fluorosulfonate de magnésium, le méthanesulfonate de calcium, le méthanesulfonate de magnésium.

**8.** Procédé selon l'une des revendications 1 à 7, caractérisé en ce que l'on utilise des mélanges de plusieurs sels de métaux alcalins ou alcalino-terreux.

**9.** Procédé selon l'une des revendications 1 à 8, caractérisé en ce que les oxacides du phosphore sont les composés à fonction acide du phosphore au degré d'oxydation 5.

**10.** Procédé selon la revendication 9, caractérisé en ce que les oxacides du phosphore V sont l'acide orthophosphorique, l'acide métaphosphorique, l'acide pyrophosphorique, les acides polyphosphoriques, les acides phosphoniques tels que l'acide (hydroxy-1 éthylidène)diphosphonique, l'acide phosphonique, l'acide éthylphosphonique, l'acide phénylphosphonique.

**11.** Procédé selon l'une des revendications 9 ou 10, caractérisé en ce que les oxacides du phosphore sont l'acide orthophosphorique, l'acide pyrophosphorique et l'acide (hydroxy-1 éthylidène)diphosphonique.

**12.** Procédé selon l'une des revendications 1 à 11, caractérisé en ce que la quantité de sel de métal alcalin ou alcalino-terreux, exprimée en rapport molaire sel de métal alcalin ou alcalino-terreux/peroxyde d'hydrogène est compris entre 0,1 % et 10 % et de préférence entre 0,5 % et 5 %.

**13.** Procédé selon l'une des revendications 1 à 11, caractérisé en ce que la quantité d'oxacide du phosphore exprimée en rapport molaire oxacide du phosphore/peroxyde d'hydrogène est comprise entre 0,1 % et 20 % et de préférence entre 0,5 % et 10 %.

**14.** Procédé selon l'une des revendications 1 à 13, caractérisé en ce que l'on utilise un rapport molaire composé phénolique de formule (I)/peroxyde d'hydrogène de 25/1 à 3/1 et de préférence de 20/1 à 4/1.

**15.** Procédé selon l'une des revendications 1 à 14, caractérisé en ce que l'on utilise comme composés phénoliques de formule (I) le phénol, l'anisole, l'orthocrésol, le paracrésol, le métacrésol, le tertiobutyl-4 phénol, le méthoxy-2 phénol, le méthoxy-4 phénol.

**16.** Procédé selon l'une des revendications 1 à 15, caractérisé en ce que la température à laquelle est conduite la réaction d'hydroxylation est comprise entre 45°C et 160°C sous pression atmosphérique.

## Claims

**1.** Process for the hydroxylation of phenols or phenol ethers of general formula (I):

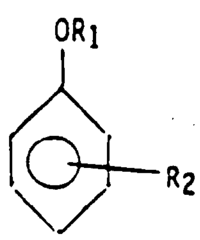

(I)

in which

- $R_1$ represents a hydrogen atom, a methyl group, an ethyl group or a phenyl group;
- $R_2$ represents a hydrogen atom, an alkyl radical having from 1 to 4 carbon atoms, an alkoxy radical having from 1 to 4 carbon atoms or a phenyl or cyclohexyl radical,

by reaction with hydrogen peroxide, characterised in that the reaction is carried out in the presence:

- of an effective amount of an alkali metal or alkaline earth metal salt of at least one protonic acid having a pKa in water of less than -0.1;
- and of an effective amount of at least one phosphorus oxyacid.

**2.** Process according to claim 1, characterised in that the protonic acids whose salts serve as catalysts are

those whose pKa in water is less than -1.

3. Process according to either of claims 1 or 2, characterised in that the alkali metal or alkaline earth metal salts of sulphuric acid, pyrosulphuric acid, perchloric acid, nitric acid, halogenosulphonic acids such as chlorosulphonic acid and fluorosulphonic acid, trifluoromethanesulphonic acid, methanesulphonic acid, ethanesulphonic acid, ethanedisulphonic acid, benzenesulphonic acid, the benzenedisulphonic acids, the toluenesulphonic acids, the naphthalenesulphonic acids and the naphthalenedisulphonic acids are used.

4. Process according to one of claims 1 to 3, characterised in that the alkali metal salts used are the neutral lithium, sodium, potassium, rubidium and cesium salts of the acids defined in claim 3, and preferably the sodium or potassium salts.

5. Process according to one of claims 1 to 4, characterised in that the alkali metal salts used are disodium sulphate, sodium perchlorate, sodium trifluoromethanesulphonate , sodium paratoluenesulphonate, sodium chlorosulphonate, sodium fluorosulphonate and sodium methanesulphonate.

6. Process according to one of claims 1 to 3, characterised in that the alkaline earth metal salts used are the neutral beryllium, magnesium, calcium,strontium and barium salts of the acids defined in claim 3, and preferably the magnesium, calcium and barium salts.

7. Process according to one of claims 1 to 3 and 6, characterised in that the alkaline earth metal salts used are calcium sulphate, magnesium sulphate, calcium perchlorate, magnesium perchlorate, calcium trifluoromethanesulphonate, magnesium trifluoromethanesulphonate, calcium paratoluenesulphonate, magnesium paratoluenesulphonate, calcium chlorosulphonate, magnesium chlorosulphonate, calcium fluorosulphonate, magnesium fluorosulphonate, calcium methanesulphonate and magnesium methanesulphonate.

8. Process according to one of claims 1 to 7, characterised in that mixtures of several alkali metal or alkaline earth metal salts are used.

9. Process according to one of claims 1 to 8, characterised in that the phosphorus oxyacids are the compounds of phosphorus with a degree of oxidation of 5 which have an acid function.

10. Process according to claim 9, characterised in that the oxyacids of phosphorus V are orthophosphoric acid, metaphosphoric acid, pyrophosphoric acid, polyphosphoric acids and phosphonic acids such as 1-hydroxyethylidenediphosphonic acid, phosphonic acid, ethylphosphonic acid and phenylphosphonic acid.

11. Process according to either of claims 9 or 10, characterised in that the phosphorus oxyacids are orthophosphoric acid, pyrophosphoric acid and 1-hydroxyethylidenediphosphonic acid.

12. Process according to one of claims 1 to 11, characterised in that the amount of alkali metal or alkaline earth metal salt expressed as the molar ratio of alkali metal or alkaline earth metal salt/hydrogen peroxide is between 0.1 % and 10 % and preferably between 0.5 % and 5 %.

13. Process according to one of claims 1 to 11, characterised in that the amount of phosphorus oxyacid expressed as a molar ratio of phosphorus oxyacid/hydrogen peroxide is between 0.1 % and 20 % and preferably between 0.5 % and 10 %.

14. Process according to one of claims 1 to 13, characterised in that a molar ratio of phenolic compound of formula (I)/hydrogen peroxide of 25/1 to 3/1 and preferably of 20/1 to 4/1 is used.

15. Process according to one of claims 1 to 14, characterised in that the phenolic compounds of formula (I) used are phenol, anisole, orthocresol, paracresol, metacresol, 4-tert-butylphenol, 2-methoxyphenol and 4-methoxyphenol.

16. Process according to one of claims 1 to 15, characterised in that the temperature at which the hydroxylation reaction is carried out is between 45°C and 160°C under atmospheric pressure.

## Patentansprüche

1. Verfahren zur Hydroxylierung von Phenolen oder Phenolethern der allgemeinen Formel (I)

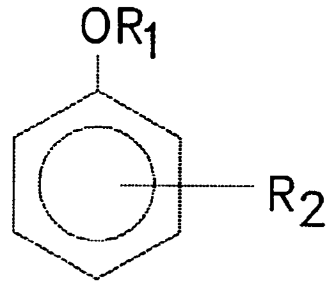

in der
- $R_1$ ein Wasserstoffatom, eine Methylgruppe, eine Ethylgruppe oder eine Phenylgruppe ist;
- $R_2$ ein Wasserstoffatom, ein Alkylradikal, das 1 bis 4 C-Atome enthält, ein Alkoxyradikal, das 1 bis 4 C-Atome enthält, ein Phenylradikal oder ein Cyclohexyl ist;

durch Reaktion mit Wasserstoffperoxid, dadurch gekennzeichnet, daß die Reaktion in Gegenwart von
- einer wirksamen Menge Alkalimetall- oder Erdalkalimetallsalzen von wenigstens einer Wasserstoffsäure, die in Wasser einen pKa-Wert kleiner als - 0,1 hat;
- und einer wirksamen Menge wenigstens einer Sauerstoffphosphorsäure

durchgeführt wird.

2. Verfahren nach Anspruch 1, dadurch gekennzeichnet, daß die Wasserstoffsäuren, deren Salze als Katalysatoren dienen, solche mit einem pKa-Wert in Wasser von weniger als - 1 sind.

3. Verfahren nach einem der Ansprüche 1 oder 2, dadurch gekennzeichnet, daß man die Salze der Alkalimetalle oder Erdalkalimetalle mit Schwefelsäure, Pyroschwefelsäure, Perchlorsäure, Salpetersäure, Halogensulfonsäuren wie Chlorsulfonsäure, Fluorsulfonsäure , Trifluormethansulfonsäure, Methansulfonsäure, Ethansulfonsäure, Ethandisulfonsäure, Benzolsulfonsäure, Benzoldisulfonsäuren, Toluolsulfonsäuren, Naphthalinsulfonsäuren und Naphthalindisulfonsäuren einsetzt.

4. Verfahren nach einem der Ansprüche 1 bis 3, dadurch gekennzeichnet, daß man als Alkalimetallsalze die Neutralsalze der in Anspruch 3 definierten Säuren mit Lithium, Natrium, Kalium, Rubidium und Caesium, vorzugsweise die Salze mit Natrium oder Kalium einsetzt.

5. Verfahren nach einem der Ansprüche 1 bis 4, dadurch gekennzeichnet, daß man als Alkalimetallsalze Dinatriumsulfat , Natriumperchlorat , Natriumtrifluormethansulfonat , Natrium-fluorsulfonat und Natriummethansulfonat einsetzt.

6. Verfahren nach einem der Ansprüche 1 bis 3, dadurch gekennzeichnet, daß man als Erdalkalimetallsalze, die Neutralsalze der in Anspruch 3 definierten Säuren mit Beryllium, Magnesium, Calcium, Strontium und Barium, vorzugsweise die Salze mit Magnesium, Calcium und Barium einsetzt.

7. Verfahren nach einem der Ansprüche 1 bis 3 und 6, dadurch gekennzeichnet, daß man als Erdalkalimetallsalze Calciumsulfat, Magnesiumsulfat, Calciumperchlorat, Magnesiumperchlorat, Calciumtrifluormethansulfonat, Magnesium-trifluormethansulfonat, Calcium-para-toluolsulfonat, Magnesium-paratoluolsulfonat, Calcium-chlorsulfonat, Magnesium-chlorsulfonat, Calcium-fluorsulfonat, Magnesium-fluorsulfonat, Calcium-methansulfonat, Magnesium-methansulfonat einsetzt.

8. Verfahren nach einem der Ansprüche 1 bis 7, dadurch gekennzeichnet, daß man die Mischung mehrerer dieser Alkali- oder Erdalkalimetallsalze verwendet.

9. Verfahren nach einem der Ansprüche 1 bis 8, dadurch gekennzeichnet, daß die Phosphorsauerstoffsäuren saure Verbindungen mit Phosphor in der Oxidationsstufe 5 sind.

10. Verfahren nach Anspruch 9, dadurch gekennzeichnet, daß die Sauerstoffsäuren des Phosphor V Orthophosphorsäure, Metaphosphorsäure, Pyrophosphorsäure, Polyphosphorsäuren, Phosphonsäuren wie 1-Hydroxyethan-1,1-diphosphonsäure, Phosphonsäure, Ethylphosphonsäure, Phenylphosphonsäure sind.

**11.** Verfahren nach einem der Ansprüche 9 oder 10, dadurch gekennzeichnet, daß die Sauerstoffsäuren des Phosphors Orthophosphorsäure, Pyrophosphorsäure und 1-Hydroxyethan-1,1-diphosphonsäure sind.

**12.** Verfahren nach einem der Ansprüche 1 bis 11, dadurch gekennzeichnet, daß die Menge der Alkali- oder Erdalkalimetallsalze, ausgedrückt als Molverhältnis von Alkali- oder Erdalkalimetallsalzen zu Wasserstoffperoxid zwischen 0,1 und 10 %, vorzugsweise zwischen 0,5 und 5 % liegt.

**13.** Verfahren nach einem der Ansprüche 1 bis 11, dadurch gekennzeichnet, daß die Menge der Phosphorsauerstoffsäure , ausgedrückt als Molverhältnis von Phosphorsauerstoffsäure zu Wasserstoffperoxid zwischen 0,1 und 20 %, vorzugsweise zwischen 0,5 und 10 % liegt.

**14.** Verfahren nach einem der Ansprüche 1 - 13, dadurch gekennzeichnet, daß man ein Molverhältnis der Phenolverbindung entsprechend Formel (I) zu Wasserstoffperoxid von 25 : 1 bis 3 : 1, vorzugsweise von 20 : 1 bis 4 : 1 einsetzt.

**15.** Verfahren nach einem der Ansprüche 1 bis 14, dadurch gekennzeichnet, daß man als Phenolverbindungen der Formel (I) Phenol, Anisol, ortho-Kresol, para-Kresol, meta-Kresol, 4-tert. Butyl-phenol, 2-Methoxy-phenol, 4-Methoxy-phenol einsetzt.

**16.** Verfahren nach einem der Ansprüche 1 bis 15, dadurch gekennzeichnet, daß die Temperatur, bei der die Hydroxylierungsreaktion durchgeführt wird, zwischen 45 und 160 °C bei Atmosphärendruck liegt.